# EUROPEAN PATENT SPECIFICATION

(11) **EP 1 813 945 B1**
(45) Date of publication and mention of the grant of the patent: **06.04.2011**
(21) Application number: 06256613.8
(22) Date of filing: 29.12.2006
(51) Int. Cl.: G01N 33/53

(54) **A method for determining the phenotype of cells**
Verfahren zur Bestimmung des Phänotyps von Zellen
Procédé de détermination du phénotype de cellules

(30) Priority: 30.12.2005 US 755611 P
(43) Date of publication of application: 01.08.2007
(73) Proprietor: Centocor Ortho Biotech Inc., Horsham, PA 19044 (US)
(72) Inventor: Popma, Sicco H., Conshohocken, PA 19428 (US)
(74) Representative: Tunstall, Christopher Stephen

(56) References cited:
- WO-A-02/074789
- ROSA DE S C ET AL: "11-Color, 13-parameter flow cytometry: Identification of human naive T cells by phenotype, function, and T-cell receptor diversity" NATURE MEDICINE, NATURE PUBLISHING GROUP, NEW YORK, NY, US, vol. 7, no. 2, February 2001 (2001-02), pages 245-248, XP002323311 ISSN: 1078-8956
- BOROWITZ MICHAEL J ET AL: "Prognostic significance of fluorescence intensity of surface marker expression in childhood B-precursor acute lymphoblastic leukemia. A Pediatric Oncology Group study" BLOOD, vol. 89, no. 11, 1997, pages 3960-3966, XP002431213 ISSN: 0006-4971
- HAMANN DORTE ET AL: "Phenotypic and functional separation of memory and effector human CD8+ T cells" JOURNAL OF EXPERIMENTAL MEDICINE, vol. 186, no. 9, 3 November 1997 (1997-11-03), pages 1407-1418, XP002431214 ISSN: 0022-1007

## Description

### FIELD OF THE INVENTION

This invention is directed to methods for the rapid, efficient and accurate determination of the phenotype of cells. In particular, this invention is directed to methods employing antibodies.

### BACKGROUND

The surface of every cell in the body is coated with proteins. These cell surface proteins have several functions. For example, the cell surface proteins may be receptors, which have the capability of selectively binding or adhering to other "signaling" molecules. Alternatively, these proteins may be functional or structural. For example, the protein may facilitate the adherence of the cell to a substrate, or the protein may facilitate the secretion of a molecule. Each cell type, for example a liver cell, has a certain combination of proteins on their surface that makes them distinguishable from other kinds of cells or cell systems.

The expression of cell surface proteins on a given cell type may vary, depending on a variety of factors. The may include, for example, changes as a result of cellular proliferation, differentiation, disease, isolation of the cell from the body and culture *in vitro.* For example, cell surface proteins have been reported to play vital roles in multiple steps of prostate cancer metastasis, such as detachment of tumor cells from the extracellular matrix, resistance of tumor cells to the detachment-induced apoptosis, adhesion of tumor cells to endothelial cells and angiogenesis to support tumor growth. There may be cell surface proteins either uniquely or differentially expressed in both epithelial and endothelial cells that are important for individual processes of prostate cancer metastasis.

Researchers have taken advantage of the biological uniqueness of cell surface proteins and chemical properties of certain compounds to tag or "mark" cells. Cells marked in this manner can readily be isolated and characterized. In many cases, a combination of multiple markers is used to identify a particular cell type...

Antibodies are used extensively as diagnostic tools in a wide array of different analyses. Monoclonal and recombinant antibodies may be used as probes to tag or mark cells. Antibody-based immunoassays are the most commonly used type of diagnostic assay and still one of the fastest growing technologies for the analysis of biomolecules. Another example with particular importance in diagnostics that has become a routine during the past decade is flow cytometric analysis. Flow cytometry is a method for quantifying components or structural features of cells primarily by optical means. Although it makes measurements on one cell at a time, it can process thousands of cells in a few seconds. Since different cell types can be distinguished by quantifying structural features, flow cytometry can be used to count cells of different types in a mixture. Flow cytometers may also be employed to select or "sort" cells based on their cell surface marker expression.

Intracellular components may also be reported by fluorescent probes, including total DNA/cell (allowing cell cycle analysis), newly synthesized DNA, specific nucleotide sequences in DNA or mRNA, filamentous actin, and any structure for which an antibody is available. Flow cytometry can also monitor rapid changes in intracellular free calcium, membrane potential, pH, or cell signaling pathways. For example, Krutzik et al (Journal of Immunology, 2005, 175: 2357-2365) state "phosphospecific flow cytometry has emerged as a powerful tool to analyze intracellular signaling events in complex populations of cells because of its ability to simultaneously discriminate cell types based on surface marker expression and measure levels of intracellular phosphoproteins. This has provided novel insights into the cell- and pathway-specific nature of immune signaling."

Flow cytometry analysis and sorting studies using monoclonal antibodies to define the surface markers on normal and neoplastic cell populations created the basis for routine clinical diagnostic assays that now range from leukemia classification to monitoring CD4 T-cell loss as HIV disease progresses.

Despite the versatility of flow cytometry, there are some drawbacks. For example, the antibodies chosen for flow cytometry reagents may not be optimal: They may not be specific, resulting in a high background staining. Furthermore, the efficiency of coupling the fluorochrome to the antibody may vary from batch to batch. Therefore, comparisons between populations of cells may be further complicated.

In addition, in order to assess multiple parameters on cell populations, a significant investment is required to purchase the required antibodies. For example, the analysis of 90 single parameters would require the purchase of 90 separate antibodies, which may amount to several tens of thousands of dollars and a large number of cells per analysis. This is problematic if the number of cells is finite and limited. Therefore, there is a significant need for cost effective, validated, reproducible reagents and antibodies for the phenotyping or analysis of cells.
Rosa et at. (Nature Medicine, 7, 245 - 248, 2001) identified human naive T cells using flow cytometry.
Borowitz et al. (Blood, 89:11, 3960-3966, 1997) used flow cytometry to measure surface membrane antigen expression in patients diagnosed with B-precursor acute lymphoblastic leukaemia.
Hamann et al. (J. Exp. Med., 186:3, 1407-1418, 1997) characterised memory and effector human CD8+ T cells using flow cytometry.
WO 02/074789 (Baylor College of Medicine) characterised HIV patients' blood monocytes using flow cytometry.

### SUMMARY

The present invention provides a kit comprising a multi-well plate containing antibodies that are lyophilised or otherwise configured for convenient transport and storage, wherein the antibodies are for detecting the panel of phenotypic markers described in Table 2.

The present invention also provides a kit for comparing the phenotypic state of a first unknown sample material with a sample material of a known phenotypic state wherein the sample material does not device from a human embryo comprising;
a. the panel of phenotypic markers described in Table 2, and
b. antibodies to detect the presence of the phenotypic markers of Table 2 in a sample material.

The present invention also provides a method for comparing the phenotypic state of a first unknown sample material with a sample material of a known phenotypic state comprising the steps of:
a. Obtaining a first unknown sample material, and;
b. Contacting the first unknown sample material with antibodies to detect the presence of the panel of phenotypic markers described in Table 2 in the first unknown sample material, and;
c. Recording the presence of phenotypic markers in the first unknown sample material, and;
d. Obtaining a sample material of a known phenotypic state, and;
e. Contacting the sample material of a known phenotypic state with said reagents to detect the presence of said panel of phenotypic markers wherein the sample material does not device from a human embryo in the sample material of a known phenotypic state, and;
f. Recording the presence of phenotypic markers in the sample material of a known phenotypic state, and;
g. Reporting the differences between the unknown sample material and the sample material of a known phenotypic state.

### BRIEF DESCRIPTION OF THE DRAWINGS

FIGURE 1: Outline of the method of the present invention.

FIGURE 2: Analysis of the phenotypic state of a population of cells using the marker reagent array shown in Table 2.

FIGURE 3: Analysis on the effects of enzymatic treatment on the phenotypic state of different samples of populations of cell X, using the marker reagent array shown in Table 2.

FIGURE 4: Comparison of the phenotypic state of a sample of cell X with a sample of human dermal fibroblsts, using the marker reagent array shown in Table 2.

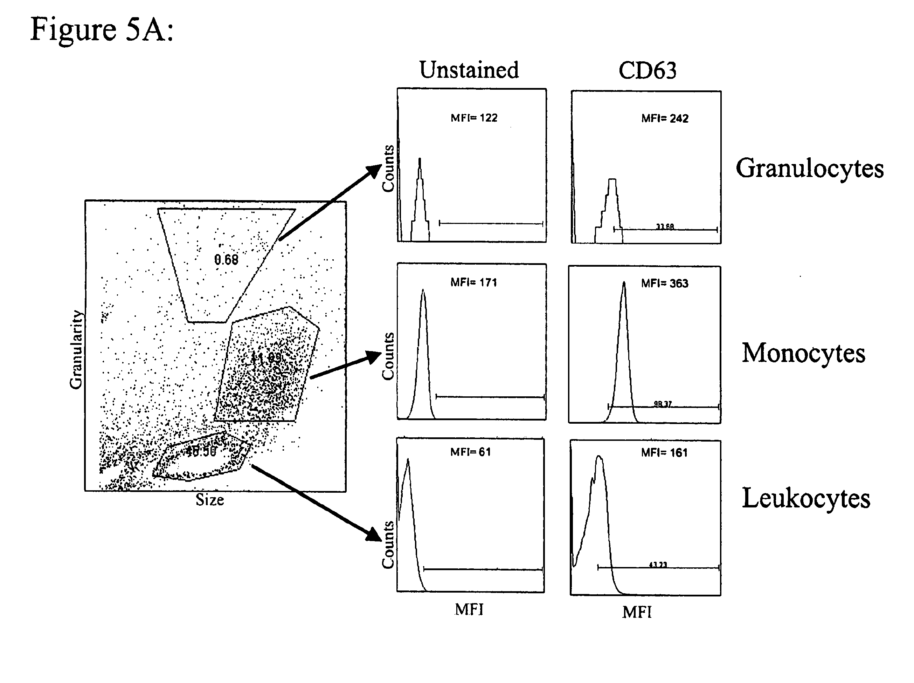

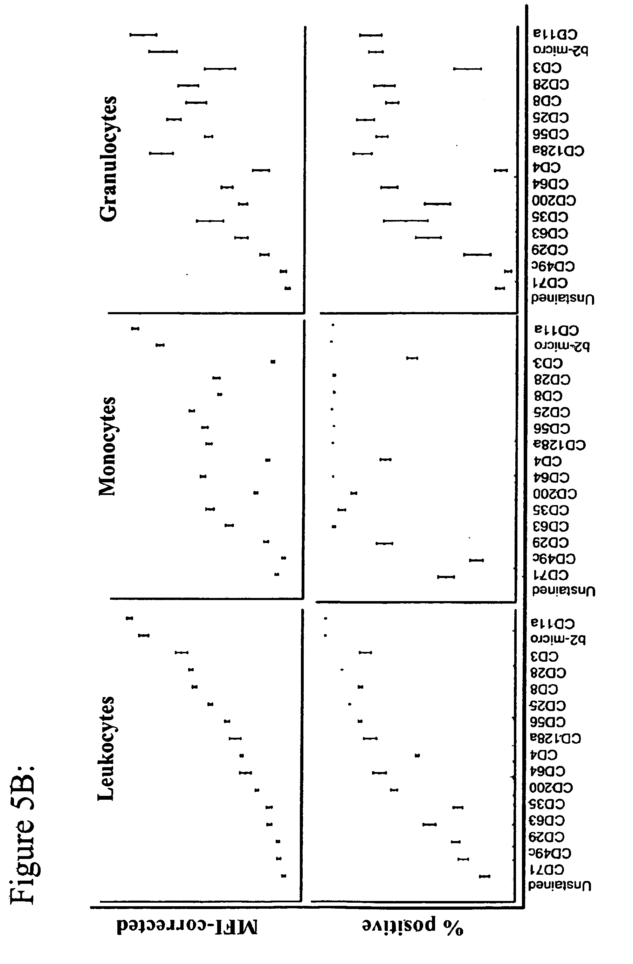

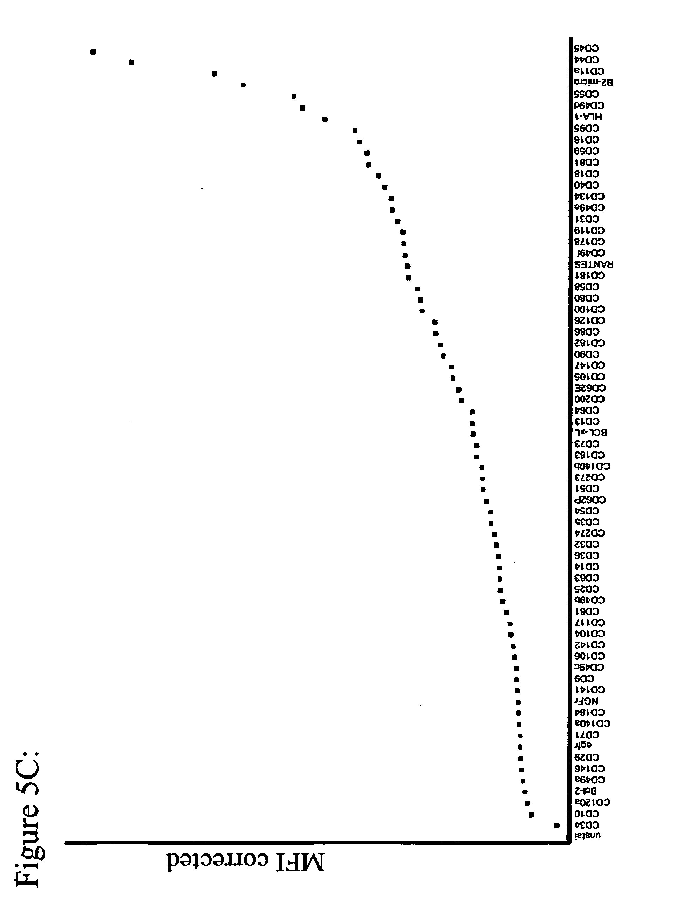

FIGURE 5: Phenotypic analysis of whole blood. Figure 5 A shows the level of expression of a selected epitope in heterogenous cell samples. Leukocyte, monocyte and granulocyte populations were selected based upon size and granularity. Percentage of positive events and Mean Fluorescence Intensity was assessed for all selected cell populations for each epitope tested. As an example the expression levels of CD63 are shown for all selected cell populations. Figure 5 B shows the percentage of positive events and expression levels of leukocytes, monocytes and granulocytes for the array of surface epitopes shown. Cell populations were selected and analyzed from three healthy donors in 11 separate tests (Mean ± SEM). Figure 5 C shows expression levels of the epitopes in the reagent array shown in table 1 for a leukocyte population taken from a healthy individual.

### DETAILED DESCRIPTION

According to the present invention, assay components and methods for the characterization of the phenotype of cells are provided. As those of ordinary skill in the art will recognize, the invention has an enormous number of applications in diagnostic assay techniques. Reagents may be prepared, for example, so as to detect or screen for any of a number of sample characteristics, pathological conditions, or reactants. The present invention provides a panel of reagents that is selected to cover various applications.

### THE REAGENTS

Reagents suitable for use in the present invention are antibodies that recognize phenotypic markers. The phenotypic markers may be proteins expressed on the surface of cells, or they may be proteins that are expressed intracellularly. Alternatively, they may be proteins that are secreted from the cell. Alternatively, the phenotypic markers may be nucleic acids, lipids, polysaccharides or any biomolecule.

Reagents suitable for use in the present invention are antibodies that specifically the phenotypic markers. In one embodiment, the reagents are labeled with a fluorescent marker. Alternatively the reagent may be intrinsically fluorescent.

Antibodies suitable for use in the present invention may be obtained from commercial sources, or they may be generated for a specific application. In one embodiment, the antibodies are labeled with a fluorescent marker. Examples of antibodies and phenotypic markers suitable for use in the present invention are shown in **Table 2**.

### THE REAGENT PANELS

The present invention provides cost effective, optimized reagents for the reproducible analysis of cells. In one embodiment, the reagent is in the form of a kit, comprising a multi-well plate containing antibodies that are lyophilized or otherwise configured allowing for convenient transport and storage. The multi-well plates may have different formats such as 6, or 8, or 12, or 24, or 96, or 384 wells and the like. The antibodies are selected according to the analysis that is to be performed. The antibodies may be selected to cover immune related epitopes. Alternatively, they may be selected to cover cancer epitopes, or stem cell epitopes. The antibodies may recognize extracellular molecules or they may recognize intracellular molecules.

The selected antibodies may be dispensed into multi-well plates according to the layouts shown in Tables 2 and 3. The layouts are unique to the specific phenotypic marker panel. Each well of the multiwell plate may contain a single antibody or multiple antibodies. The selected antibodies are provided in bulk quantities and dispensed into a large number of plates to reduce variation between samples. The antibodies may be stored in the plates in solution prior to analysis. Alternatively, the antibodies may be stored lyophilized and re-constituted prior to analysis.

### THE METHOD

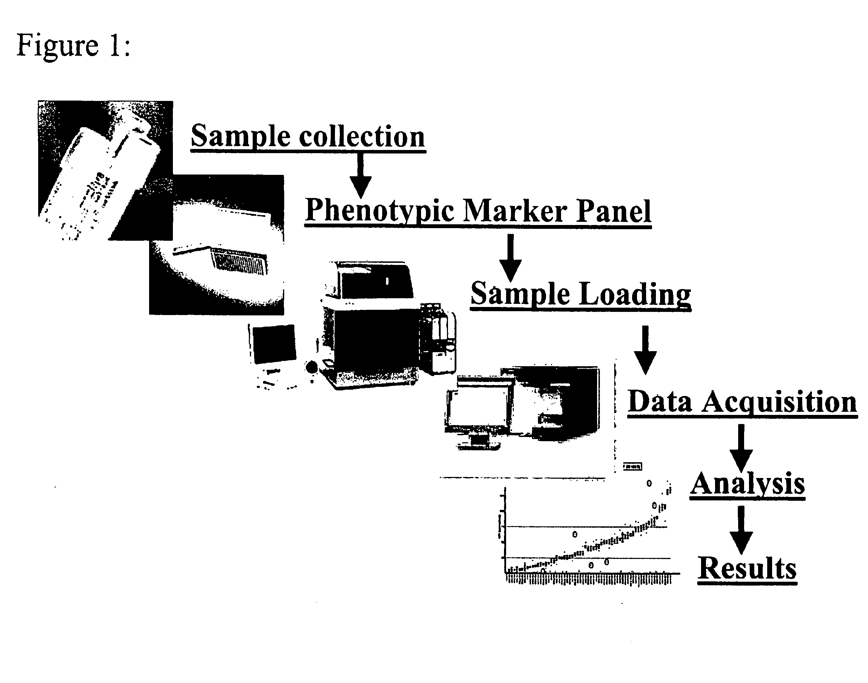

In one embodiment, the method of the present invention comprises a system that carries out the steps of collecting samples material of an unknown phenotypic state and collecting sample material of a known phenotypic state. The sample material may comprise, for example, cells, blood, or any biological sample. The system analyses the sample materials to identify patterns epitope markers in the sample material of known phenotypic state that is used as a comparator for the sample material of unknown phenotypic state. The identity of the epitopes in the identified pattern may be known. Alternatively, more than one epitope in the identified pattern may be unknown. An outline of the method of the present invention is shown in **Figure 1**.

For example, the methods of the present invention may be used for quality control purposes. In this case, the sample material of known phenotypic state may be obtained from a control population of cells. This sample material may then be used as a comparator for the sample material of unknown phenotypic state that consists of populations of cell obtained from production runs. Changes from the patterns of epitope markers in the sample material of known phenotypic may be used to accept or reject production runs. An example of this is shown in **Example 1.**

Alternatively, the present invention may be used to report changes in the pattern of epitope markers in a population of cells following treatment with a factor or pharmaceutical agent. An example of this is shown in **Example 2.** Alternatively, the present invention may be used to report changes in the pattern of epitope markers between distinct populations of cells. An example of this is shown in **Example 3**.

In one embodiment, the present invention may also report changes in the pattern of epitope markers in distinct populations of cells in whole blood. The cell populations may be from a sample of blood from one patient. Alternatively, samples from more patient may be used.

The methods of the present invention may be employed to analyze one sample or, alternatively, more than one sample. In one embodiment, the high-throughput analysis of a large number of samples may be carried out. The steps of the method of the present invention may be carried manually. Alternatively, at least one step may be automated. Multiple copies of a phenotypic marker reagent plate may be made and used to analyze samples.

Analysis of the samples may be performed by any suitable platform known to those of ordinary skill in the art. Such platforms may include, for example, a flow cytometry apparatus, a mass spectrometry apparatus, or a fluorescent microscope, and the like.

Flow cytometry apparatuses rely upon flow of cells or other particles in a liquid flow stream in order to determine one or more characteristics of the cells under investigation. Further, the flow cytometry apparatus is useful for identifying the presence of certain cells or particles of interest, enumerating those cells or particles and, in some instances, providing a sorting capability so as to be able to collect those cells or particles of interest. In a typical flow cytometry apparatus, a fluid sample containing cells is directed through the flow cytometry apparatus in a rapidly moving liquid stream so that each cell passes serially, and substantially one at a time, through a sensing region. Cell volume may be determined by changes in electrical impedance as each cell passes through the sensing region. Similarly, if an incident beam of light is directed at the sensing region, the passing cells scatter such light as they pass there through. This scattered light serves as a function of cell shape and size, index of refraction, opacity, roughness and the like. Further, fluorescence emitted by labeled cells, or autofluorescent cells, which have been excited as a result of passing through the excitation energy of the incident light beam is detectable for identification of cells having fluorescent properties. After cell analysis is performed by the flow cytometry apparatus, those cells that have been identified as having the desired properties may be sorted if the apparatus has been designed with such capability.

Representative flow cytometry apparatuses are described in U.S. Pat. Nos. 3,826,364 and 4,284,412, and in the publication by Herzenberg et al., "Fluorescence-activated Cell Sorting," Sci. Am. 234 (3): 108, 1976.

The present invention is further illustrated, but not limited by, the following examples.

### EXAMPLES

### EXAMPLE 1: QUALITY CONTROL; RELEASE CRITERIA

*Master plate preparation:* An antibody template was designed to monitor changes of expression across a wide array of epitopes in order to establish consistency of the cell product between production and expansion runs. Antibodies were selected and dispensed into a multiwell plate according to the layout shown in **Table 2.** Antibodies were purchased from the sources shown in **Table 1.** Antibodies were diluted in PBS into a 96-well master plate and stored in a refrigerator prior to analysis. For each assay 10µl of master antibody solution was transferred from the master plate to a new 96 well plate as such that a copy was created. The array contained phycoerythrin fluorescein labeled antibodies against one specific marker per well. This eliminated the need for compensation of the machine, simplified the assay and reduced operator bias. In addition, the staining protocol was designed to minimize the number of cells needed for the assay and eliminate steps, such as washing, that can cause errors.

*Assay:* A vial of cells were thawed, washed and finally diluted to 2x10⁵ cells per ml in PBS and a 100µl sample was transferred to each well (20,000 cells/well) containing diluted antibodies. The plate was left to incubate for 30 minutes at 4°C. Next 100µl fixation buffer (Cytofix, BD catalog no. 554655) was added and left for 15 minutes. A FACSCalibur machine with a high through put system was setup for analysis and 10,000 events per well were acquired.

*Data analysis:* For this study the acquired data was exported to an analysis software program and the Mean Fluorescent Intensity (MFI) of each sample well was assessed. The MFI of the unstained sample was subtracted from each sample MFI and this delta MFI was plotted for each epitope. This was repeated eight times in independent experiments creating an average expression pattern for our cell product.

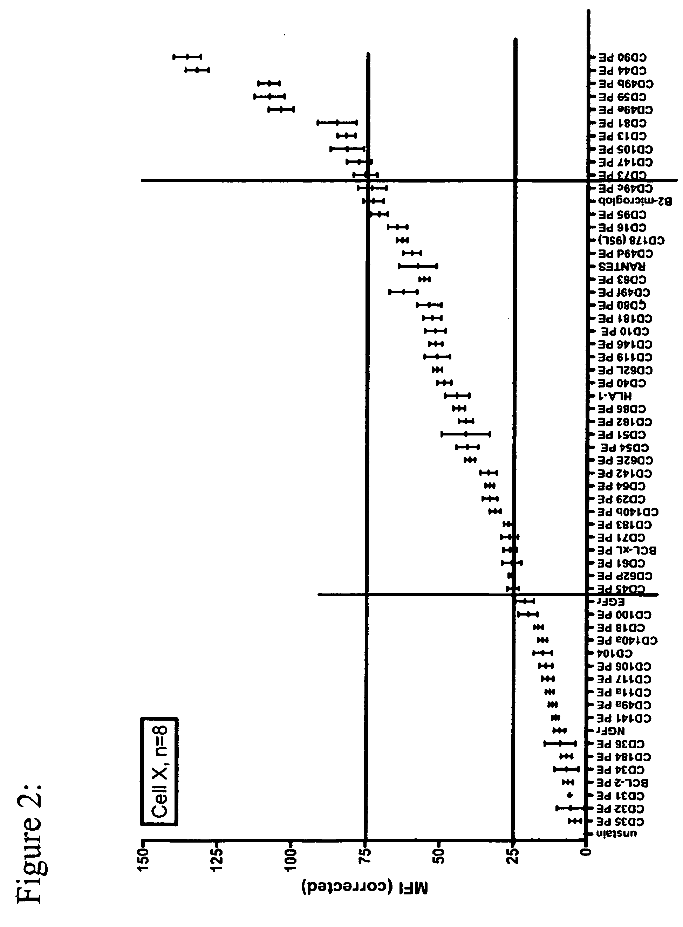

*Results:* Cells obtained from different production/expansion runs were routinely stained using the 96-well plate array and the MFI were plotted and compared against the pattern of epitope markers obtained from the sample material of known phenotypic state. The results are shown in **Figure 2**.

### EXAMPLE 2: COMPARISON OF DISSOCIATION REAGENTS

*Method:* The methods and reagent panel used for this assay is the same as described in **Example 1.** Cells were grown in 3 separate culture flasks under identical conditions. These cultures were harvested each with a different dissociation enzyme mix. The cells were then stained using out 96-well method and the mean fluorescence intensity was measured for each parameter and compared to the pattern of epitope markers obtained from the sample material of known phenotypic state.

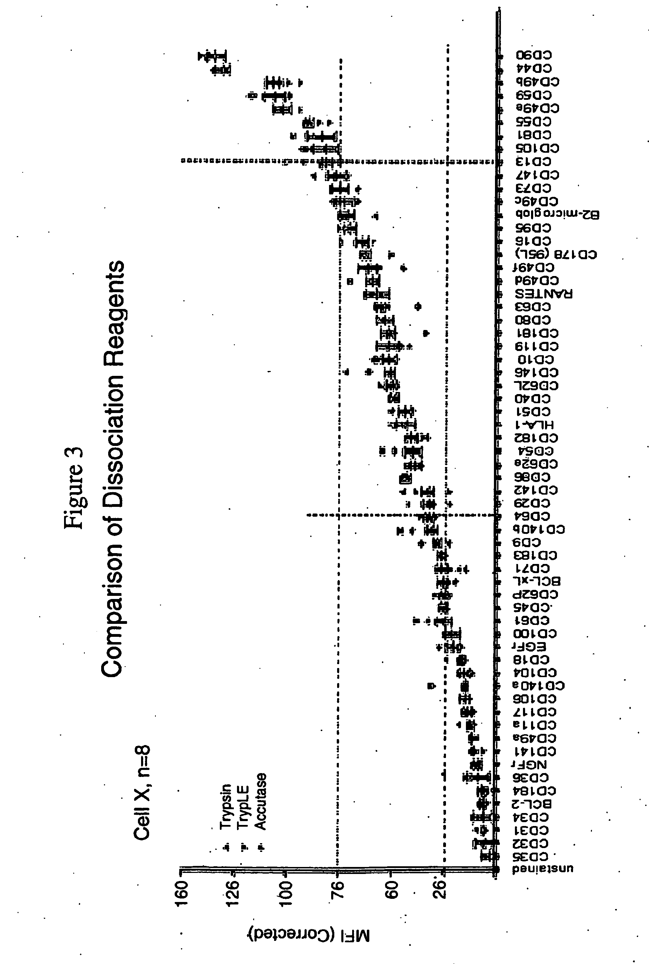

*Results:* Each enzyme mix showed different results when compared to the pattern of epitope markers obtained from the sample material of known phenotypic state. Mixture C (Accutase) seems to affect the expression of certain phenotypic marker more than the standard mix A (Trypsin) and B (TriplE). For example the adhesion marker CD49f and the signaling molecule CD63 show lower expression levels after the harvesting procedure, which may affect the cell product's function. Results are shown in **Figure 3**.

*Conclusion:* This method allows for a rapid assessment of a wide array of proteins expressed on the cell surface. Changes to the phenotypic state that may occur following treatment with enzymes can readily be assessed by this method. The availability of a master plate allows for a quick assessment of phenotypic changes across a wide array of phenotypic markers due to treatment of the cells.

### EXAMPLE 3: COMPARISON OF THE PHENOTYPIC STATE OF TWO DISTINCT CELL POPULATIONS

*Method:* The methods and reagent panel used for this assay were the same as described in **Example 1**. In this assay expression levels epitopes tested by the master plate on a different homogenous cell culture. The cell culture of interest was grown to confluency and harvested according our standard protocol. Both cell cultures were then stained in the 96-well master plate and analyzed according the methods described earlier. The phenotypic state of the cell populations were compared and key differences were identified.

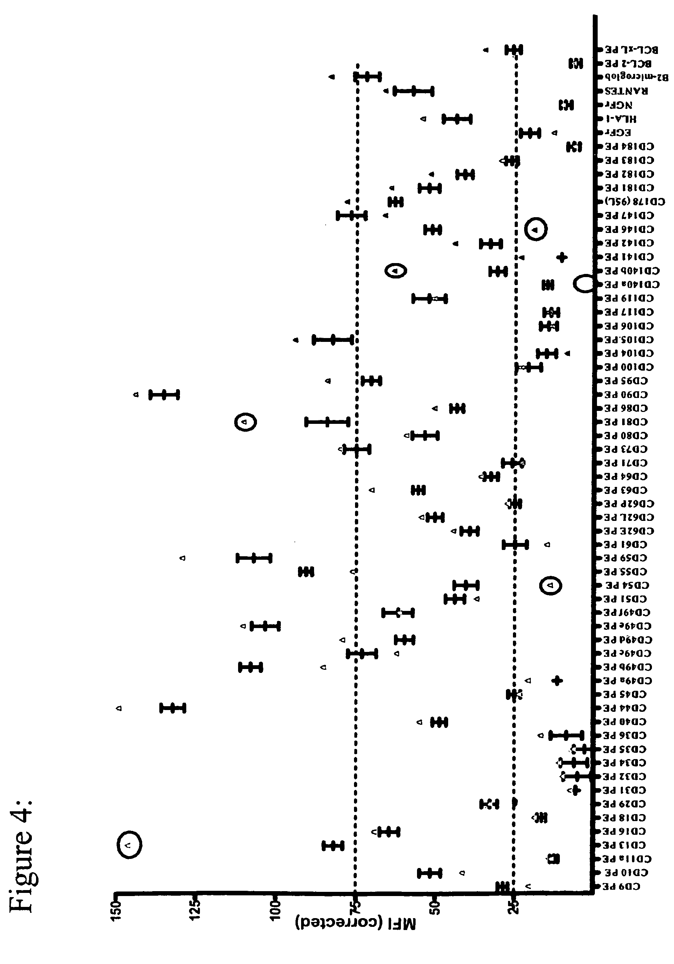

*Results:* The two cell types have similar expression levels for most of the epitopes except for example CD11a and CD54. These epitopes were differentially expressed. We selected an arbitrary cut-off for significance at a delta of 25 MFI compared to the sample material of known phenotypic state. The results are shown in **Figure 4**.

*Conclusion:* We showed expression differences between two cell types in our panel. These differences can be further explored and can become important starting points of further research especially if the phenotypic marker changes can be linked to in-vivo efficacy differences.

### EXAMPLE 4: PHENOTYPIC ANALYSIS OF WHOLE BLOOD

*Method:* The methods and reagent panel used for this assay were the same as described in **Example 1**. Heparinized peripheral blood was obtained from healthy volunteers and white blood cells were isolated. The White blood cells were washed and suspended in PBS and transferred to the 96-well staining plate (50,000 cells per well) and data was acquired according to the standard protocol. Data analysis, however, is different since the sample contains multiple cell populations each with their specific expression pattern for each epitope. Analyzing the cells as a bulk may obscure these effects. Therefore three main populations were selected based upon size and granularity and each was analyzed for percentage positive events within the gate and expression levels of each epitope tested (**Figure 5** **A,B and C**).

*Results:* Using the 96-well platform we able to quickly assess expression levels of our array of epitopes for three distinct populations in peripheral blood (**Figure 5**).

*Conclusion:* The 96 well plate can also be used for heterogeneous cell population. However the data processing and mining becomes significantly more complex. Novel analysis methods are in development to simplify the analysis, which is especially important when this method is expanded to multiple color flow cytometry. This method may be used to determine the pattern of epitope markers in easy accessible peripheral blood samples for healthy (non-disease phenotype) individuals and use this a comparator for cytomes obtained from individuals with a particular disease. Differences can be exploited for, but not limited to, development of diagnostics, measure drug efficacy and selection of treatment regiment.

**TABLE 1: PHENOTYPIC MARKERS DISCLOSURE**

| **CD identification** | **MOLECULE** | **Gene Name** | **Fluorescence** | **company** |
|---|---|---|---|---|
| CD3 | CD3/T-cell antigen receptor (TCR) | CD3G/Z | PE | Caltag |
| CD4 | OKT4, Leu 3a, T4 | CD4 | PE | BD Pharmingen |
| CD5 | T1, Leu1 | CD5 | PE | BD PharMingen |
| CD7 | Leu 9, 3A1, gp40, T cell leukemia antigen | CD7 | PE | BD Pharmingen |
| CD8 | MHC class I receptor | CD8 | PE | BD PharMingen |
| CD9 | type III transmembrane protein (platelet aggregation/activation) | CD9 | PE | BD PharMingen |
| CD10 | CALLA, membrane metallo-endopeptidase | MME | PE | BD Pharmingen |
| CD11a | alphaL; LFA-1, gp180/95 | ITGAL | PE | BD Pharmingen |
| CD13 | Aminopeptidase N, APN, gp150, EC 3.4.11.2 | ANPEP | PE | BD Pharmingen |
| CD15 | fucosyltransferase 4 (alpha (1,3) fucosyltransferase, myeloid-specific) | FUT4 | PE | BD PharMingen |
| CD16 | Fc gamma R III | FCGR3A | PE | Caltag |
| CD16a | Fc gamma R IIIa | FCGR3A | PE | BD Pharmingen |
| CD18 | β2-Integrin chain, macrophage antigen 1 (mac-1) | ITGB2 | PE | BD Pharmingen |
| CD19 | B4, associated with CD21, CD81, CD225, Leu-13, Lyn, Fyn, Vav, P13-kinase | CD19 | PE | BD PharMingen |
| CD20 | membrane-spanning 4-domains, subfamily A, member 1 | MS4A1 | PE | BD PharMingen |
| CD21 | C3d receptor,CR2, gp140; EBV receptor | CR2 | PE | BD Pharmingen |
| CD22 | Bgp135; BL-CAM, Siglec2 | CD22 | FITC | BD Pharmingen |
| CD25 | Interleukin-2 receptor (IL-2R, inflammatory response) | IL2RA | PE | BD PharMingen |
| CD29 | Integrin β1 chain; platelet GPlla; VLA (CD49) beta-chain | ITGB1 | PE | BD Pharmingen |
| CD30 | tumor necrosis factor receptor superfamily, member 8. KI-1 | TNFRSF8 | PE | BD PharMingen |
| CD31 | PECAM-1; platelet GPIIa'; endocam | PECAM1 | PE | BD Pharmingen |
| CD32 | Fcgamma receptor type II (FcgRII), gp40 | FCGR2A | PE | BD Pharmingen |
| CD33 | gp67 | CD33 | PE | BD PharMingen |
| CD34 | My10, gp105-120 | CD34 | PE | BD PharMingen |
| CD35 | C3b/C4b receptor; complement receptor type 1 (CR1) | CR1 | PE | BD Pharmingen |
| CD36 | platelet GPIV, GPIIIb, OKM-5 antigen | CD36 | PE | BD Pharmingen |
| CD38 | T10; gp45, ADP-ribosyl cyclase | CD38 | PE | BD Pharmingen |
| CD40 | Bp50, TNF Receptor 5 | TNFRSF5 | PE | BD PharMingen |
| CD44 | Pgp-1; gp80-95, Hermes antigen, ECMR-III and HUTCH-I. | CD44 | PE | BD PharMingen |
| CD45 | LCA, B220, protein tyrosine phosphatase, receptor type, C | PTPRC | PE | BD Pharmingen |
| CD49a | Integrin a1 chain, very late antigen, VLA 1a | ITGA1 | PE | BD Pharmingen |
| CD49b | Integrin a2 chain, VLA-2-alpha chain, platelet gpla | ITGA2 | PE | BD Pharmingen |
| CD49c | Integrin a3 chain, VLA-3 alpha chain | ITGA3 | PE | BD Pharmingen |
| CD49d | Integrin a4 chain" VLA-4-alpha chain | ITGA4 | PE | BD Pharmingen |
| CD49e | Integrin a5 chain" VLA-5 alpha chain | ITGA5 | PE | BD Pharmingen |
| CD49f | Integrin a6 chain" VLA-6 alpha chain, platelet gplc | ITGA6 | PE | BD Pharmingen |
| CD50 | ICAM-3, intercellular adhesion molecule 3 | ICAM3 | fitc | BD Pharmingen |
| CD51 | Integrin alpha chain, vitronectin receptor a chain | ITGAV | PE | BD Pharmingen |
| CD54 | ICAM-1, intercellular adhesion molecule 1 | ICAM1 | PE | BD Pharmingen |
| CD55 | decay-accelerating factor (DAF, prevent cell damage) | DAF | PE | BD PharMingen |
| CD56 | Neural cell adhesion molecule 1 (NCAM1), NKH-1 (NK cell marker) | NCAM1 | PE | Caltag |
| CD58 | Lymphocyte function-associated antigen-3 (LFA-3, interacts with CD2 during cell adhesion) | CD58 | PE | BD Pharmingen |
| CD59 | MACIF, MIRL, P-18, protectin | CD59 | PE | BD Pharmingen |
| CD61 | Glycoprotein IIIa, beta3 integrin | ITGB3 | PE | BD Pharmingen |
| CD62E | E-selectin, LECAM-2, ELAM-1 | SELE | PE | BD Pharmingen |
| CD62L | L-selectin, LAM-1, Mel-14 | SELL | PE | Caltag |
| CD62P | P-selectin, granule membrane protein-140 (GMP-140) | SELP | PE | BD Pharmingen |
| CD63 | LIMP, gp55, LAMP-3 neuroglandular antigen, granulophysin | CD63 | PE | BD Pharmingen |
| CD64 | FcgR1, FcgammaR1 (IgG-receptor) | FCGR1A | PE | Caltag |
| CD69 | Early T-cell activation antigen | CD69 | PE | BD PharMingen |
| CD71 | Transferrin receptor | TFRC | PE | BD Pharmingen |
| CD73 | Ecto-5'-nucleotidase | NT5E | PE | BD Pharmingen |
| CD79a | CD79A antigen (immunoglobulin-associated alpha), MB1 | CD79A | PE | BD PharMingen |
| CD79b | CD79B antigen (immunoglobulin-associated beta), B29 | CD79B | PE | BD PharMingen |
| CD80 | B7-1; BB1 | CD80 | PE | BD Pharmingen |
| CD81 | Target of an antiproliferative antibody (TAPA-1); M38 | CD81 | PE | BD Pharmingen |
| CD83 | HB15 | CD83 | PE | BD Pharmingen |
| CD86 | B7-2; B70 | CD86 | PE | Caltag Laboratories |
| CD87 | plasminogen activator, urokinase receptor (PLAUR), uPAR | PLAUR | PE | BD PharMingen |
| CD88 | C5a-receptor | C5R1 | PE | BD Pharmingen |
| CD90 | Thy-1 | THY1 | PE | BD PharMingen |
| CD95 | APO-1, Fas, TNFRSF6 | TNFRSF6 | PE | BD Pharmingen |
| CD100 | SEMA4D | SEMA4D | PE | Serotec |
| CD103 | integrin, alpha E (human mucosal lymphocyte antigen 1; alpha polypeptide) | ITGAE | PE | BD PharMingen |
| CD104 | Integrin beta 4 subunit, TSP-1180 | ITGB4 | PE | BD Pharmingen |
| CD105 | Endoglin | ENG | PE | Caltag |
| CD106 | VCAM-1 (vascular cell adhesion molecule-1), INCAM-110 | VCAM1 | PE | BD Pharmingen |
| CD114 | G-CSFR, HG-CSFR, CSFR3 | CSF3R | PE | BD Pharmingen |
| CD117 | SCFR, c-kit, stem cell factor receptor | KIT | PE | BD Biosciences |
| CD119 | IFN gamma receptor alpha chain (GIR-208) | IFNGR1 | PE | BD Pharmingen |
| CD120b | TNFRI; TNFRp55 | TNFRSF1B | PE | BD Pharmingen |
| CD126 | IL-6 receptor alpha chain | IL6R | PE | BD Pharmingen |
| CD134 | Tumor Necrosis Factor receptor 4, OX40 (promotes expression of BCL2/BCL-xL) | TNFRSF4 | PE | Caltag |
| CD135 | fms-related tyrosine kinase 3 | FLT3 | PE | BD PharMingen |
| CD140b | b-platelet derived growth factor (PDGF) receptor | PDGFRB | PE | BD Pharmingen |
| CD141 | Thrombomodulin (TM), fetomodulin | THBD | PE | BD Pharmingen |
| CD142 | Tissue factor, thromboplastin, coagulation factor III | F3 | PE | BD Pharmingen |
| CD146 | Muc 18, MCAM, Mel-CAM, s-endo | MCAM | PE | BD Pharmingen |
| CD147 | Basigin, M6, extracellular metalloproteinase inducer (EMMPRIN) | BSG | PE | Serotec |
| CD151 | Platelet-endothelial tetra-span antigen (PETA)-3 | CD151 | PE | BD Pharmingen |
| CD152 | Cytolytic T lymphocyte-associated antigen (CTLA-4) | CTLA4 | PE | BD PharMingen |
| CD163 | M130, GHI/61, RM3/1 | CD163 | PE | BD PharMingen |
| CD164 | MUC-24, MGC 24, multi-glycosylated core protein 24 | CD164 | PE | BD Pharmingen |
| CD165 | AD2, gp 37 | | PE | BD Pharmingen |
| CD178 | FAS ligand, CD95 ligand | TNFSF6 | PE | Caltag Laboratories |
| CD181 | CXCR1 CDw128a: IL-8 receptor alpha, | IL8RA | PE | BD Pharmingen |
| CD182 | CXCR2 | IL8RB | PE | BD Pharmingen |
| CD183 | CXCR3 chemokine receptor, G protein-coupled receptor 9 | CXCR3 | PE | BD Pharmingen |
| CD184 | CXCR4 chemokine receptor, Fusin | CXCR4 | PE | BD Pharmingen |
| CD200 | OX2 (regulates Mf activity) | CD200 | PE | BD PharMingen |
| CD273 | B7DC, PDL2 | PDCD1 LG2 | PE | BD Pharmingen |
| CD274 | B7H1, PDL1 | PDCD1LG1 | PE | BD Pharmingen |
| CD275 | B7H2, ICOSL | ICOSL | | |
| CD276 | B7H3 | N/A | | |
| CD277 | BT3.1 | BTN3A1 | | |
| CD278 | ICOS | ICOS | | |
| CD279 | PD1 | PDCD1 | | |
| CD280 | ENDO180 | MRC2 | | |
| CD281 | TLR1 | TLR1 | | |
| CD282 | TLR2 | TLR2 | | |
| CD283 | TLR3 | TLR3 | | |
| CD284 | TLR4 | TLR4 | | |
| CD289 | TLR9 | TLR9 | | |
| CD292 | BMPR1A | BMPR1A | | |
| CDw293 | BMPR1B | BMPR1B | | |
| CD294 | CRTH2 | GPR44 | | |
| CD295 | LeptinR | LEPR | | |
| CD296 | ART1 | ART1 | | |
| CD297 | ART4 | DO | | |
| CD298 | Na+/K+ -ATPase b3 | ATP1B3 | | |
| CD299 | DCSIGN-related | CD209L | | |
| CD300a | CMRF35H | | | |
| CD300c | CMRF35A | | | |
| CD300e | CMRF35L1 | | | |
| CD301 | MGL, CLECSF14 | CLECSF14 | | |
| CD302 | DCL1 | N/A | | |
| CD303 | BDCA2 | CLECSF7 | | |
| CD304 | BDCA4, Neuropilin 1 | NRP1 | | |
| CD305 | LAIR1 | LAIR1 | | |
| CD306 | LAIR2 | LAIR2 | | |
| CD307 | IRTA2 | N/A | | |
| CD309 | VEGFR2, KDR | KDR | | |
| CD312 | EMR2 | EMR2 | | |
| CD314 | NKG2D | KLRK1 | | |
| CD315 | CD9P1 | PTGFRN | | |
| CD316 | EWI2 | IGSF8 | | |
| CD317 | BST2 | BST2 | | |
| CD318 | CDCP1 | N/A | | |
| CD319 | CRACC | SLAMF7 | | |
| CD320 | 8D6A | N/A | | |
| CD321 | JAM1 | F11R | | |
| CD322 | JAM2 | JAM2 | | |
| CD324 | E-Cadherin | CDH1 | | |
| CDw325 | N-Cadherin | CDH2 | | |
| CD326 | Ep-CAM | TACSTD1 | | |
| CDw327 | siglec6 | SIGLEC6 | | |
| CDw328 | sigiec7 | SIGLEC7 | | |
| CDw329 | sigiec9 | SIGLEC9 | | |
| CD331 | FGFR1 | FGFR1 | | |
| CD332 | FGFR2 | FGFR2 | | |
| CD333 | FGFR3 | FGFR3 | | |
| CD334 | FGFR4 | FGFR4 | | |
| CD335 | NKp46 | NCR1 | | |
| CD336 | NKp44 | NCR2 | | |
| CD337 | NKp30 | NCR3 | | |
| CDw338 | ABCG2, BCRP | ABCG2 | | |
| CD339 | Jagged-1 | JAG1 | | |
| ABCG2 | | | | |
| alkaline phosphatase | | | | |
| BCL-xL | BCL2like-1 | BCL2L1 | PE | Chemicon |
| bCL-2 | B-cell CLL/lymphoma-2 | BCL2 | PE | BD Pharmingen |
| CK 5 | Cytokeratin 5 | | | |
| CK 6 | Cytokeratin 6 | | | |
| CK 7 | Cytokeratin 7 | | | |
| CK 8 | Cytokeratin 8 | | | |
| CK 10 | Cytokeratin 10 | | | |
| CK 13 | Cytokeratin 13 | | | |
| CK 14 | Cytokeratin 14 | | | |
| CK 15 | Cytokeratin 15 | | | |
| CK 16 | Cytokeratin 16 | | | |
| CK 18 | Cytokeratin 18 | | | |
| CK 19 | Cytokeratin 19 | | | |
| EGFr | Epidermal growth factor receptor (growth and differentiation) | EGFR | PE | BD PharMingen |
| HGF-R | Hepatocyte growth factor receptor | HGFR | | |
| NGFr | NGFR (p75) | NGFR | PE | BD Pharmingen |
| RANTES | CCL5 (chemokine (C-C motif) ligand 5 | CCL5 | PE | Caltag |
| b2-microglobulin | Associates with HLA class I antigen complex | B2M | PE | BD Pharmingen |
| HLA-ABC | MHC Class I ABC | | PE | BD Pharmingen |
| HLA-DP | MHC II HLA-DP beta-1 | HLA-DPB1 | PE | Serotec |
| HLA DR | | | PE | BD Pharmingen |
| SSEA-1 | Embryonic Stem Cell Antigen 1 | SSEA1 | | |
| SSEA-3 | Embryonic Stem Cell Antigen 3 | SSEA3 | | |
| SSEA-4 | Embryonic Stem Cell Antigen 4 | SSEA4 | | |

**TABLE 2: A STEM CELL PHENOTYPIC MARKER ARRAY PLATE OF THE INVENTION**

| | 1 | 2 | 3 | 4 | 5 | 6 | 7 | 8 | 9 | 10 | 11 | 12 |
|---|---|---|---|---|---|---|---|---|---|---|---|---|
| A | Unstain | Unstain | Unstain | Unstain | Unstain | Unstain | CD9 | CD10 | CD11a | CD13 | CD14 | CD16 |
| B | CD18 | CD25 | CD29 | CD31 | CD32 | CD34 | CD35 | CD36 | CD40 | CD44 | CD45 | CD49a |
| C | CD49b | CD49c | CD49d | CD49e | CD49f | CD51 | CD54 | CD55 | CD58 | CD59 | CD61 | CD62E |
| D | CD62L | CD62P | CD63 | CD64 | CD71 | CD73 | CD80 | CD81 | CD86 | CD90 | CD95 | CD100 |
| E | CD104 | CD105 | CD106 | CD117 | CD119 | CD120a | CD126 | CD134 | CD140a | CD140b | CD141 | CD142 |
| F | CD146 | CD147 | CD178 | CD181 | CD182 | CD183 | CD184 | CD200 | CD273 | CD274 | β2-microglobulin | HLA-ABC |
| G | HLA-DP | EGFR | NGFR | RANTES | CD3 | CD4 | CD7 | CD15 | CD19 | CD20 | CD21 | CD22 |
| H | CD38 | CD50 | CD62L | CD83 | CD88 | CD112 | CD114 | CD151 | CD164 | CD165 | CD172 | HLA-DR |

**TABLE 3: AN EXAMPLE OF AN ONCOLOGY PHENOTYPIC MARKER ARRAY PLATE**

| | 1 | 2 | 3 | 4 | 5 | 6 | 7 | 8 | 9 | 10 | 11 | 12 |
|---|---|---|---|---|---|---|---|---|---|---|---|---|
| A | Unstain | Unstain | Unstain | Unstain | Unstain | Unstain | CD3 | CD4 | CD5 | CD7 | CD8 | CD9 |
| B | CD10 | CD11a | CD13 | CD14 | CD15 | CD16 | CD18 | CD19 | CD20 | CD22 | CD25 | CD29 |
| C | CD30 | CD31 | CD32 | CD33 | CD34 | CD35 | CD36 | CD38 | CD40 | CD44 | CD45 | CD49a |
| D | CD49b | CD49c | CD49d | CD49e | CD49f | CD51 | CD54 | CD55 | CD56 | CD58 | CD59 | CD61 |
| E | CD62E | CD62L | CD62P | CD63 | CD64 | CD69 | CD71 | CD73 | CD79a | CD79b | CD80 | CD81 |
| F | CD86 | CD87 | CD90 | CD95 | CD100 | CD103 | CD104 | CD105 | CD106 | CD117 | CD119 | CD120a |
| G | CD126 | CD134 | CD135 | CD140a | CD140b | CD141 | CD142 | CD146 | CD147 | CD152 | CD163 | CD178 |
| H | CD181 | CD182 | CD183 | CD184 | CD200 | CD273 | CD274 | b2-micorglobulin | EGFR | HLA-ABC | HLA-1 (DP) | RANTES |

## Claims

1. A kit comprising a multi-well plate containing antibodies that are lyophilised or otherwise configured for convenient transport and storage, wherein the antibodies are for detecting the panel of phenotypic markers described in Table 2.

2. A kit for comparing the phenotypic state of a first unknown sample material with a sample material of a known phenotypic state comprising;
a. the panel of phenotypic markers described in Table 2, and
b. antibodies to detect the presence of the phenotypic markers of Table 2 in a sample material.

3. The kit of claim 1 or 2 wherein the antibodies are lyophilised antibodies.

4. The kit of any of claims 1 to 3 wherein the antibodies are labelled with a fluorescent marker.

5. A method for comparing the phenotypic state of a first unknown sample material with a sample material of a known phenotypic state comprising the steps of:
a. Obtaining a first unknown sample material, and;
b. Contacting the first unknown sample material with antibodies to detect the presence of the panel of phenotypic markers described in Table 2 in the first unknown sample material, and;
c. Recording the presence of phenotypic markers in the first unknown sample material, and;
d. Obtaining a sample material of a known phenotypic state, and;
e. Contacting the sample material of a known phenotypic state wit sai reagents to detect the presence of said panel of phenotypic markers of Table 2 in the sample material of a known phenotypic state, and;
f. Recording the presence of phenotypic markers in the sample material of a known phenotypic state, and;
g. Reporting the differences between the unknown sample material and the sample material of a known phenotypic state.

6. The method of claim 5 for use in quality control purposes.

7. The kit of any of claims 1-4 or the method of claim 5 or claim 6 wherein the sample material is cells.

## Patentansprüche

1. Kit, umfassend eine Multi-Napf-Platte, enthaltend Antikörper, die lyophilisiert oder anderweitig für einen bequemen Transport und Lagerung konfiguriert sind, wobei die Antikörper zum Nachweisen der Sammlung von Phänotyp-Markern dient, die in Tabelle 2 beschrieben sind.

2. Kit zum Vergleichen des Phänotyp-Zustands eines ersten unbekannten Probenmaterials mit einem Probenmaterial eines bekannten Phänotyp-Zustands, umfassend
a) die Sammlung der in Tabelle 2 beschriebenen Phänotyp-Marker und
b) Antikörper zum Nachweis des Vorhandenseins der Phänotyp-Marker aus Tabelle 2 in einem Probenmaterial.

3. Kit nach Anspruch 1 oder 2, wobei die Antikörper lyophilisierte Antikörper sind.

4. Kit nach einem der Ansprüche 1-3, wobei die Antikörper mit einem fluoreszierenden Marker markiert sind.

5. Verfahren zum Vergleichen des Phänotyp-Zustands eines ersten unbekannten Probenmaterials mit einem Probenmaterial eines bekannten Phänotyp-Zustands, umfassend die Schritte:
a) Erhalt eines ersten unbekannten Probenmaterials und
b) In-Kontakt-Bringen des ersten unbekannten Probenmaterials mit Antikörpern zum Nachweisen des Vorhandenseins der Sammlung von Phänotyp-Markern, die in Tabelle 2 beschrieben sind, in dem ersten unbekannten Probenmaterial und
c) Aufzeichnen des Vorhandenseins von Phänotyp-Markern in dem ersten unbekannten Probenmaterial und
d) Erhalt eines Probenmaterials eines bekannten Phänotyp-Zustands und
e) In-Kontakt-Bringen des Probenmaterials eines bekannten Phänotyp-Zustands mit den Reagenzien zum Nachweis des Vorhandenseins der Sammlung von Phänotyp-Markern aus Tabelle 2 in dem Probenmaterial eines bekannten Phänotyp-Zustands und
f) Aufzeichnen des Vorhandenseins von Phänotyp-Markern in dem Probenmaterial eines bekannten Phänotyp-Zustands und
g) Ausweisen der Unterschiede zwischen dem unbekannten Probenmaterial und dem Probenmaterial eines bekannten Phänotyp-Zustands.

6. Verfahren nach Anspruch 5, zur Verwendung zu Qualitätskontrollzwecken.

7. Kit nach einem der Ansprüche 1-4 oder Verfahren nach Anspruch 5 oder Anspruch 6, wobei das Probenmaterial Zellen ist.

## Revendications

1. Kit comprenant une plaque multi-puits contenant des anticorps qui sont lyophilisés ou autrement configurés pour un transport et stockage pratiques, où les anticorps sont pour la détection du panneau de marqueurs phénotypiques décrits dans le Tableau 2.

2. Kit pour comparer un état phénotypique d'un premier matériau d'échantillon inconnu avec un matériau d'échantillon d'un état phénotypique connu comprenant:
a. le panneau de marqueurs phénotypiques décrits dans le Tableau 2, et
b. des anticorps pour détecter la présence des marqueurs phénotypiques du Tableau 2 dans un matériau d'échantillon.

3. Kit selon la revendication 1 ou 2, dans lequel les anticorps sont des anticorps lyophilisés.

4. Kit selon l'une quelconque des revendications 1 à 3, dans lequel les anticorps sont marqués par un marqueur fluorescent.

5. Procédé de comparaison de l'état phénotypique d'un premier matériau d'échantillon inconnu avec un matériau d'échantillon d'un état phénotypique connu comprenant les étapes de:
a. obtenir un premier matériau d'échantillon inconnu, et
b. mettre en contact le premier matériau d'échantillon inconnu avec des anticorps pour détecter la présence du panneau de marqueurs phénotypiques décrits dans le Tableau 2 dans le premier matériau d'échantillon inconnu, et
c. enregistrer la présence de marqueurs phénotypiques dans le premier matériau d'échantillon inconnu; et
d. Obtenir un matériau d'échantillon d'un état phénotypique connu, et
e. mettre en contact le matériau d'échantillon d'un état phénotypique connu avec lesdits réactifs pour détecter la présence dudit panneau de marqueurs phénotypiques du Tableau 2 dans le matériau d'échantillon d'un état phénotypique connu, et
f. enregistrer la présence de marqueurs phénotypiques dans le matériau d'échantillon d'un état phénotypique connu, et
g. signaler les différences entre le matériau d'échantillon inconnu et le matériau d'échantillon d'un état phénotypique connu.

6. Procédé selon la revendication 5 pour utilisation à des fins de contrôle de qualité.

7. Kit selon l'une quelconque des revendications 1 à 4 ou le procédé selon la revendication 5 ou la revendication 6, dans lequel le matériau d'échantillon sont des cellules.
